(19) Europäisches Patentamt
European Patent Office
Office européen des brevets

(11) Veröffentlichungsnummer: **0 114 954**
**B1**

(12) # EUROPÄISCHE PATENTSCHRIFT

(45) Veröffentlichungstag der Patentschrift: **19.02.86**

(51) Int. Cl.[4]: **C 07 H 15/04**, C 07 H 15/26, A 23 L 1/236

(21) Anmeldenummer: **83111300.6**

(22) Anmeldetag: **11.11.83**

(54) **Derivate und Reduktionsprodukte der D-Glucopyranosyl-alpha(1-5)-D-arabonsäure, deren Herstellung und Verwendung.**

(30) Priorität: **28.12.82 DE 3248404**

(43) Veröffentlichungstag der Anmeldung: **08.08.84 Patentblatt 84/32**

(45) Bekanntmachung des Hinweises auf die Patenterteilung: **19.02.86 Patentblatt 86/8**

(84) Benannte Vertragsstaaten: **AT CH DE FR GB LI NL SE**

(56) Entgegenhaltungen:
**FR - A - 2 310 354**
**US - A - 2 190 377**

**BULLETIN DE LA SOC. CHIM. FR., 1959, Seiten 1353-1362, J. DUBOURG et al.: "Oxydation des hexoses réducteurs par l'oxygène en milieu alcalin"**

(73) Patentinhaber: **Süddeutsche Zucker-Aktiengesellschaft, Maximilianstrasse 10, D-6800 Mannheim 1 (DE)**

(72) Erfinder: **Lichtenthaler, Frieder W., Prof. Dr., Bergstrasse 15, D-6109 Mühltal (DE)**
Erfinder: **Klimesch, Roger G., Dipl.-Ing., Georg-Fröba-Strasse 43, D-6146 Alsbach-Hähnlein (DE)**

(74) Vertreter: **Körber, Wolfhart, Dr. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22 (DE)**

EP 0 114 954 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Derivate und Reduktionsprodukte der D-Glucopyranosyl-α(1→5)-D-arabonsäure, die der allgemeinen Formel

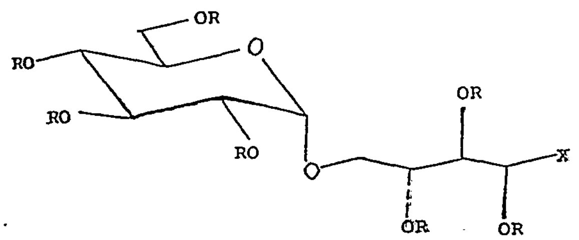

entsprechen. In dieser Formel steht X für eine

−COOAlkali-,
−COOAlkyl-,
$-CONH_2$- oder
$-CH_2OH$-Gruppe

und R bedeutet ein Wasserstoffatom oder einen Acylrest, wie einen Acetyl- oder Benzoylrest. Die Arabonsäurekette kann auch zu einem Lacton ringgeschlossen sein. Zu den genannten Derivaten gehören u.a. die Alkalisalze, die Alkylester, das Amid und das Lacton der D-Glucopyranosyl-α-(1→5)-D-arabonsäure, sowie der D-Glucopyranosyl-α(1→5)-D-arabinit.

Die Abkömmlinge der D-Glucopyranosyl-α(1→5)-D-arabonsäure, die der oben angeführten allgemeinen Formel entsprechen, stellen neue Verbindungen dar. Sie finden u.a. als Zwischenprodukte für chemische Synthesen, beispielsweise zur Herstellung von oberflächenaktiven Stoffen, technische Anwendung. Der D-Glucopyranosyl-α(1→5)-D-arabinit kann aufgrund seiner Süsskraft, wie weiter unten näher erläutert wird, in fester oder flüssiger Form – auch im Gemisch mit künstlichen Süssstoffen oder auch mit nährenden süss schmeckenden Kohlenhydraten – als Zuckeraustauschstoff vor allem für Diabetiker und Adipöse verwendet werden.

Das Ausgangsmaterial für die Herstellung der Verbindungen der oben angegebenen allgemeinen Formel bildet die Isomaltulose, das ist die D-Glucopyranosyl-α(1→6)-D-fructose der weiter unten angegebenen Formel (1), die durch eine Oxidation in alkalischer Lösung mit Luft oder Sauerstoff übergeführt wird in die Salze der D-Glucopyranosyl-α(1→5)-D-arabonsäure, durch deren Weiterverarbeitung vermittels Veresterung, Amidierung und Behandlung mit sauren, ringschliessenden Mitteln die entsprechenden Ester, das Amid und das γ-Lacton erhalten werden, während der genannte D-Glucopyranosyl-α(1→5)-arabinit aus einem der erwähnten Alkylester bzw. dem vorerwähnten Lacton oder auch deren O-Acylierungsprodukten durch bestimmte Reduktionsverfahren gewonnen werden.

Die O-Acylderivate, insbesondere die O-Acetyl- und O-Benzoylderivate der vorstehend angeführten Arabonsäureabkömmlinge können durch Behandeln der jeweiligen nicht-O-acylierten Ausgangsverbindungen mit Acylierungsmitteln in wasserfreien organischen Lösungsmitteln erhalten werden.

Die Abläufe der genannten Umsetzungen und Weiterverarbeitungen sowie die dabei entstehenden Produkte sind in dem nachstehenden Formelschema dargestellt.

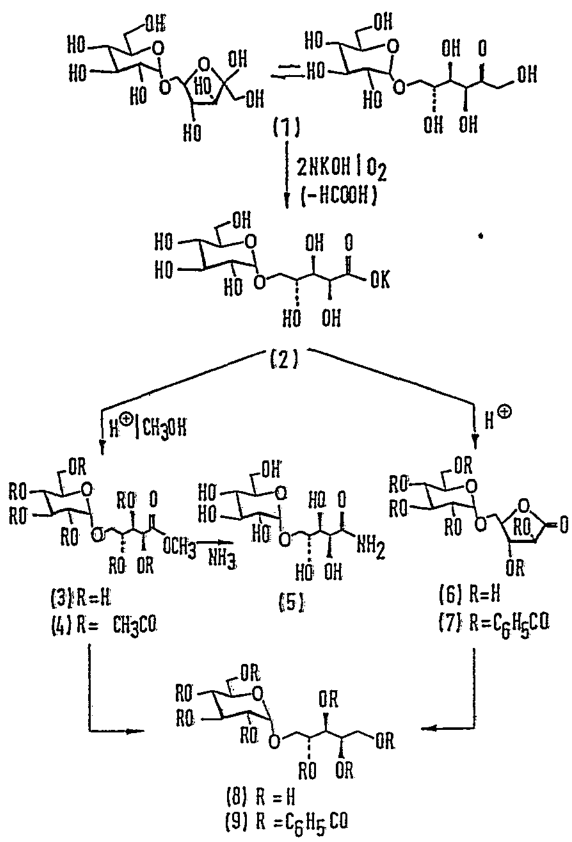


An sich ist die Oxidation reduzierender Zucker (Hexosen, Pentosen) in alkalischem Medium mit reinem Sauerstoff oder Luft aus J. Stanek, M. Cerny, J. Kocourek und J. Pacak, «The Monosaccharides», Academic Press, New York, 1963, S. 138 ff. und der dort zitierten Literatur bekannt; man erhält hierbei das Salz der um ein Kohlenstoffatom ärmeren Aldonsäure. Im Fall der Ketohexosen scheint die Reaktion komplexer zu verlaufen; so bildet L-Sorbose neben L-Xylonsäure beträchtliche Mengen der 2-Keto-L-gulonsäure, wie der US-PS 2 190 377 zu entnehmen ist, während D-Fructose neben D-Arabonat und Ameisensäure weitere Oxidationsprodukte, z.B. Glykolsäure, Glycerinsäure, Erythronsäure und Milchsäure bildet, wie die Angaben in Bull. Soc. Chim. Fr., 1959, S. 1353 ff. ausweisen.

Demgegenüber führt die Luftoxidation von Isomaltulose, d.i. die D-Glucopyranosyl-α(1→6)-D-fructose der Formel (1), ausserordentlich glatt und problemlos zum Glucopyranosyl-α(1→5)-D-arabonat der Formel (2), wenn man 5 bis 20%ige Lösungen von Isomaltulose in 2N Kalilauge bei Raumtemperatur mit Luft behandelt, z.B. die Lösung stark rührt oder die Lösung mit Luft oder Sauerstoff begast.

Die Isolierung der gebildeten Glucopyranosyl-α(1→5)-D-arabonsäure erfolgt in Form ihres Kaliumsalzes und zwar durch Neutralisation des überschüssigen Kaliumhydroxids mit einer Carbon- oder Mineralsäure und Kristallisation aus dem Reaktionsgemisch durch Zugabe von Methanol.

Die Ausbeuten an Produkt der Formel (2) liegen durchweg über 90% (vgl. Beispiel 1: 96%) und waren in anderen Fällen praktisch quantitativ. Ausser der im Verhältnis 1:1 zwangsläufig gebildeten Ameisensäure sind weitere Oxidationsprodukte, wie z.B. die in Analogie zur Fructose-oxidation erwarteten Glycosyl-α(1→4)-erythronsäure oder Glucosyl-α(1→3)-glycerinsäure, im Reaktionsgemisch praktisch nicht vorhanden; nur in mit hoher Konzentration durchgeführten Chromatogrammen lassen sich Spuren (Mengen unter 0,5%) anderer, nicht näher identifizierter Produkte nachweisen.

Die Darstellung von Derivaten der Glucopyranosyl-α(1→5)-arabonsäure aus dem Salz der Formel (2), wie z.B. dem Methylester der Formel (3) und dem Lacton der Formel (6), ist unter den üblichen Bedingungen (Mineralsäure/Methanol bzw. wässerige Mineralsäure) in nur mässigen Ausbeuten erreichbar, da die Glycosidbindung säurelabil ist und eine partielle Aufspaltung in Glucose und D-Arabinit eintritt. Die genannte Darstellung ist jedoch unter Verwendung eines sauren Ionenaustauschers und Einhaltung spezieller Bedingungen möglich (vgl. Beispiele 2 und 6), wodurch Ausbeuten von 85 bzw. 74% erzielbar sind.

Der Methylester der Formel (3) lässt sich durch Behandeln mit methanolischem Ammoniak in praktisch quantitativer Ausbeute in das schön kristallisierte Glucopyranosyl-α(1→5)-D-arabonsäureamid der Formel (5) überführen.

Die Reduktion des Methylesters der Formel (3) oder seines Heptaacetats der Formel (4) mit komplexen Metallhydriden, wie z.B. $NaBH_4$ oder $LiAlH_4$, oder mit atomarem Wasserstoff führt zum D-Glucopyranosyl-α(1→5)-D-arabinit der Formel (8), der als solcher in kristalliner Form mit Schmp. 156-158° C und $[\alpha]_D^{20}$ = +98,5° (c = 1, Wasser) charakterisiert werden kann, bzw. der in Form des Octabenzoats (Formel 9) erhalten werden kann.

Ebenso wie der Methylester liefert auch das Lacton der Formel (6) oder dessen Hexabenzoat bei Reduktion der Carboxylgruppe den Glucopyranosyl-α(1→5)-arabinit der Formel (8) in Ausbeuten über 85%.

Alle erstmals dargestellten Verbindungen, das sind die Produkte der Formel (2) bis (9), wurden mikroanalytisch und chromatographisch einhellig als Reinsubstanzen charakterisiert; ihre Konstitution und Konfiguration wurde durch 300 MHz-$^1$H-NMR-Spektren sowie anhand $^{13}$C-NMR- und massenspektroskopischer Daten zweifelsfrei bewiesen.

Wie im vergleichenden Geschmackstest mit jeweils 10 bis 15 Personen ermittelt wurde, beträgt die Süsskraft von Glucopyranosyl-α(1→5)-arabinit 45% der von Saccharose. Die Süsskraft wurde gegen 7 bis 8%ige wässerige Saccharoselösungen sowie gegen solche von Glucopyranosyl-α(1→6)-D-mannit im Dreieckstest bestimmt.

Zur Erhöhung der Süsskraft von Glucopyranosyl-α(1→5)-arabinit auf die von Saccharose oder auf eine höhere Süsskraft kann dieser Arabinit in fester Form mit künstlichen Süssstoffen, wie Benzoesäuresulfimid, Cyclohexylsulfamat oder Phenylalaninasparaginsäuremethylester vermischt oder instantisiert werden. Mit künstlichen Süssstoffen aufgesüsste Glucopyranosyl-α(1→5)-arabinit-Lösungen können zusammen getrocknet (Sprühtrocknung, Walzentrocknung, Gefriertrocknung usw.) oder direkt so verwendet werden.

Ebenso kann erfindungsgemäss Glucopyranosyl-α(1→5)-arabinit in fester oder flüssiger Form mit anderen nährenden, süss schmeckenden Kohlenhydraten, z.B. Fructose, Xylit, Sorbit, im Falle von Fructose z.B. im Gewichtsverhältnis von 1:1, vermischt werden, um die Süsskraft dieses Gemisches annähernd auf die von Saccharose zu bringen.

Bei der Zubereitung von Speisen und Getränken im Haushalt, z.B. beim Backen, Einmachen, Gelieren und bei der Zubereitung von Getränken, sowie bei der industriellen Fertigung von Nahrungs-, Genussmitteln und Getränken kann Glucopyranosyl-α(1→5)-arabinit allein oder als Glucopyranosyl-α(1→5)-arabinit-Süssungsmittel-Gemisch wie normaler Zucker (Rüben- oder Rohrzucker) verwendet werden. Wegen aller dieser Eigenschaften soll Glucopyranosyl-α(1→5)-arabinit als Zuckeraustauschstoff verwendet werden, der vor allem für Diabetiker und für Adipöse geeignet ist.

Die Erfindung wird anhand der folgenden Beispiele näher erläutert.

*Beispiel 1*

*Kalium-D-Glucopyranosyl-α(1→5)-D-arabonat (Formel 2)*

In einem 1 l-Kolben wird eine Lösung von 35,0 g (102,3 mmol) Isomaltulose (Formel 1) in 100 ml Wasser mit einer Lösung von 20 g Kaliumhydroxid in ebenfalls 100 ml Wasser vermischt und anschliessend 8 bis 16 h im Luft- oder Sauerstoffstrom oxidiert. Zur besseren Vermischung der wässerigen Lösung mit dem Sauerstoff wird während dieser Zeit mit einem Turborührer (16000 Upm) gerührt. Nach Beendigung der Reaktion wird die Lösung durch vorsichtige Zugabe einer Säure (z.B. Essigsäure) oder durch Einrühren eines stark sauren Ionenaustauschers, z.B. Amberlite®IR-120, $H^+$-Form, auf pH 7,5 gebracht und zu einem Sirup eingeengt, der beim Behandeln mit Methanol kristallisiert. Umkristallisation aus dem gleichen Lösungsmittel (2,5 l) liefert 36,0 g (96%) Glucopyranosyl-arabonat der Formel (2) in Form farbloser Nadeln vom Schmp. 172-174° C, $[\alpha]_D^{20}$ = +87,8° (c = 1, Wasser).

$^1$H-NMR (300 MHz, $D_2O$): δ = 3,42 (t, 1H, 4'-H), 3,58 (dd, 1H, 2'-H), 3,62-4,04 (m, 9H, 2-H, 3-H, 4-H, 5-$H_2$, 3'-H, 5'-H, 6'-$H_2$), 4,97 (d, 1H, 1'H), $J_{1',2'}$ = 3,5, $J_{2',3'}$ = $J_{3',4'}$ = $J_{4',5'}$ = 9,5 Hz.

$^{13}$C-NMR (75 MHz, $D_2O$): δ = 61,41 (t, C-6'), 69,42 (t, C-5), 70,36 (d, C-4'), 70,50 (d, C-4),

72,37 (d, C-2'), 72,43 (d, C-2), 72,63 (d, C-3 und C-5'), 73,98 (d, C-3'), 98,98 (d, C-1'), 179,98 (s, C-1).

MS (FC): m/e = 405 (100%, $M^{+}$+ K).

$C_{11}H_{19}O_{11}K \cdot H_2O$ (384,4)

| | | |
|---|---|---|
| Berechnet: | C 34,37 | H 5,51% |
| Gefunden: | C 34,35 | H 5,45% |

*Beispiel 2*

*D-Glucopyranosyl-α(1→5)-methyl-D-arabonat (Formel 3)*

In einem 1 l-Dreihalskolben mit Rührer und Trockenrohr werden 10,0 g (27,3 mmol) Glucopyranosylarabonat-Kalium (Formel 2, Produkt von Beispiel 1) in 600 ml absolutem Methanol suspendiert, mit 4 ml Orthoameisensäuretrimethylester und 20,0 g gut mit Wasser und Methanol gewaschenem und anschliessend im Vakuum bei 75° C Badtemperatur getrocknetem stark saurem Ionenaustauscher (Amberlite®IR-120, $H^{+}$-Form) versetzt und bei Raumtemperatur reagieren gelassen. Nach ca. 3 d war chromatographisch kein Edukt mehr nachweisbar. Der Ionenaustauscher wird abfiltriert, das Lösungsmittelvolumen auf etwa 150 ml eingeengt und die so erhaltene übersättigte Lösung über Nacht bei +5° C aufbewahrt. Die erhaltenen Kristalle werden abgesaugt und gut mit kaltem Methanol gewaschen: 7,95 g (85%) Methylester als farblose Nadeln vom Schmp. 178-179° C, $[\alpha]_D^{20}$ = +105,0° (c = 1, $H_2O$).

$^1$H-NMR (300 MHz, $D_2O$): δ = 3,43 (t, 1H, 4'-H), 3,58 (dd, 1H, 2'-H), 3,64-3,79 (m, 4H, 5-$H_2$, 3'-H), 3,81 (s, 3H, $OCH_3$), 3,83-3,99 (m, 3H, 5'-H, 6'-$H_2$), $J_{2,3}$ = 1,8, $J_{3,4}$ = 9,4, $J_{1',2'}$ = 3,7, $J_{2',3'}$ = 9,8, $J_{3',4'}$ = 9,4 Hz.

$^{13}$C-NMR (75 MHz, $D_2O$): δ = 53,60 (q, $CH_3$), 61,38 (t, C-6'), 69,25 (t, C-5), 69,59 (d, C-4), 70,41 (d, C-4'), 71,57 (d, C-2), 72,29 (d, C-2'), 72,38 (d, C-5'), 72,62 (d, C-3), 73,95 (d, C-3'), 99,07 (d, C-1'), 176,01 (s, C-1).

MS (FD): M+e = 343 (100%, $M^{+}$+1), 311 (45%, M−$CH_3OH^{+}$ + 1).

$C_{12}H_{22}O_{11}$ (342,3)

| | | |
|---|---|---|
| Berechnet: | C 42,10 | H 6,48% |
| Gefunden: | C 42,11 | H 6,40% |

*Beispiel 3*

*2,3,4,6-Tetra-O-acetyl-D-glucopyranosyl-α(1→5)-methyl-2,3,4-tri-O-acetyl-D-arabonat (Formel 4)*

In einem 100 ml-Dreihalskolben mit Rührer, Tropftrichter und Trockenrohr werden 1,6 g (4,67 mmol) Glucopyranosylarabonsäure-methylester der Formel (3) in 25 ml absolutem Pyridin gelöst und bei 0° C im Verlauf von 30 min mit 16 ml Acetanhydrid versetzt. Anschliessend lässt man 18 h bei Raumtemperatur stehen und rührt sodann zur Aufarbeitung in 150 ml Eiswasser. Extraktion mit Dichlormethan (3 × 75 ml), Waschen der vereinigten organischen Phasen mit 2N HCl, gesättigter $NaHCO_3$-Lösung und Wasser liefert nach Trocknen ($Na_2SO_4$) und Eindampfen im Vakuum das Heptaacetylderivat der Formel (4) als Sirup, der bislang nicht kristallisierte: 2,96 g (quantitativ), $[\alpha]_D^{24}$ = +110,8° (c = 1, $CHCl_3$).

$^1$H-NMR (300 MHz, $CDCl_3$): δ = um 2,0 (7s, 21H, 7 Acetyl-$CH_3$), 3,68 (dd, 1H, 5-$H_a$), 3,75 (dd, 1H, 5-$H_b$), 3,76 (s, 3H, $OCH_3$), 3,98 (ddd, 1H, 5'-H), 4,08 (dd, 1H, 6-$H_a$) 4,25 (dd, 1H, 6'-$H_b$), 4,85 (dd, 1H, 2'-H), 5,05 (dd, 1H, 4'-H), 5,10 (d, 1H, 1'-H), 5,20 (ddd, 1H, 4-H), 5,30 (d, 1H, 2-H), 5,44 (dd, 1H, 3'-H), 5,63 (dd, 1H, 3-H); $J_{2,3}$ = 2,2, $J_{3,4}$ = 8,9, $J_{4,5a}$ = 5,3, $J_{4,5b}$ = 3,6, $J_{5a,5b}$ = 11,6, $J_{1',2'}$ = 3,7, $J_{2',3'}$ = 10,2, $J_{3',4'}$ = 9,5, $J_{4'5'}$ = 10,2, $J_{5',6'a}$ = 4,4, $J_{5',6'b}$ = 2,3, $J_{6'a,6'b}$ = 12,4 Hz.

MD (FD): m/e = 637 (100% $M^{+}$ + 1).

$C_{26}H_{36}O_{18}$ (636,6)

| | | |
|---|---|---|
| Berechnet: | C 49,06 | H 5,70% |
| Gefunden: | C 48,94 | H 5,65% |

*Beispiel 4*

*D-Glucopyranosyl-α(1→5)-D-arabonsäureamid (Formel 5)*

In eine auf 0° C gekühlte Lösung von 2,0 g (5,9 mmol) Methyl-glucopyranosylarabonat der Formel (3) (Produkt von Beispiel 2) in 16 ml Methanol wird trockenes Ammoniak bis zur Sättigung eingeleitet. Nach 1,5 h bei 0° C lässt man auf Raumtemperatur kommen und dampft im Vakuum zur Trockene ein. Der so erhaltene sirupöse Rückstand kristallisiert aus absolutem Methanol (35 ml): 1,9 g (98%) Amid der Formel (5) als farblose Nadeln vom Schmp. 170° C (unter Zersetzung), $[\alpha]_D^{20}$ = +75,0° (c = 1, $H_2O$).

$^1$H-NMR (300 MHz, $D_6$-DMSO): δ = 3,05-3,18 (m, 2H, 2'-H, H'-H), 3,37-3,69 (m, 7H, 4-H, 5-$H_2$, 3'-H, 5'-H, 6'-$H_2$), 3,72 (dd, 1H, 3-H), 4,10 (d, 1H, 2-H), 4,38 (s, 1H, OH), 4,4 (d, 1H, OH), 4,59 (d, 1H, OH), 4,65 (d, 2H, 1'-H, OH), 4,77 (d, 1H, OH), 4,83 (d, 1H, OH), 5,14 (d, 1H, OH), 7,14 (d, 2H, $NH_2$); $J_{2;OH\text{-}2}$ = 6,1, $J_{3,OH\text{-}3}$ = 5,0, $J_{2,3}$ = 0, $J_{3,4}$ = 10,1, $J_{1',2'}$ = 3,7, $J_{NH_2(gem)}$ = 18,9 Hz.

MS (FD): m/e = 328 (100%, $M^{+}$ + 1), 311 (40%, M−$NH_2^{+}$ + 1).

$C_{11}H_{21}N_1O_{10}$ (327,3)

| | | | |
|---|---|---|---|
| Berechnet: | C 40,37 | H 6,47 | N 4,28% |
| Gefunden: | C 40,28 | H 6,46 | N 4,21% |

*Beispiel 5*

*D-Glucopyranosyl-α(1→5)-D-arabonsäure-γ-lacton (Formel 6)*

2,0 g (1,37 mmol) Glucopyranosylarabonat-Kalium (Formel 2, Produkt von Beispiel 1) werden in einem 100 ml-Zweihalskolben mit Rührer in 25 ml Wasser gelöst und nach Zugabe von 4,0 g mit Wasser und Methanol gewaschenem, stark saurem Ionenaustauscher (Amberlite®IR-120, $H^{+}$-Form) für 12 h bei 75° C gerührt. Filtration und Einengen in Vakuum lieferte einen chromatographisch einheitlichen Sirup, der über eine Kieselgel-Säule (100 g) durch Elution mit Aceton/Wasser (16:1) gereinigt wurde: 1,25 g (74%) Lacton der Formel (6), $[\alpha]_D^{20}$ = +92,5° (c = 1, $H_2O$).

$^1$H-NMR (60 MHz, $D_2O$): δ = 3,4-4,3 (m, 11H, 2-H, 3-H, 4-H, 5-$H_2$, 2'-H, 3'-H, 4'-H, 5'-H, 6'-$H_2$), 4,90 (d, 1H, 1'-H); $J_{1',2'}$ = 3,4 Hz.

MS (FD): m/e = 311 (100%, $M^+ + 1$).

$C_{11}H_{18}O_{10}$ (310,3)

| | | |
|---|---|---|
| Berechnet: | C 42,58 | H 5,85% |
| Gefunden: | C 42,49 | H 5,80% |

*Beispiel 6*

*5-O-(2,3,4,6-Tetra-O-benzoyl-α-D-glucopyranosyl)-2,3-di-O-benzoyl-D-arabonsäure-γ-lacton (Formel 7)*

In einem 100 ml-Dreihalskolben mit Rührer, Tropftrichter und Trockenrohr werden 700 mg (2,26 mmol) Glucopyranosyl-arabonsäurelacton der Formel (6) in 12 ml absolutem Pyridin gelöst, auf 0° C gekühlt, mit 5 ml frisch destilliertem Benzoylchlorid versetzt und über Nacht reagieren gelassen. Hydrolyse, Extraktion mit Dichlormethan (3 × 40 ml), Waschen der vereinigten organischen Phasen mit 2N HCl, Wasser, gesättigter $NaHCO_3$-Lösung und Wasser, Trocknen ($Na_2SO_4$) und Einengen liefert einen Sirup, der an Kieselgel durch Elution mit Dichlormethan/Äthylacetat (40:1) gereinigt wird: 1,8 g (75%) Hexabenzoat der Formel (7) als farbloser Sirup, $[\alpha]_D^{20}$ = +22,8° (c = 1, $CHCl_3$).

$^1$H-NMR (300 MHz, $CDl_3$): δ = 4,00 (dd, 1H, 5a-H), 4,33 (dd, 1H, 5b-H), 4,55 (dd, 1H, 6'a-H), 4,66-4,78 (m, 3H, 4-H, 5'-H, 6'b-H), 5,44 (d, 1H, 1'-H), 5,45 (dd, 1H, 2'-H), 5,77 (t, 1H, 4'-H), 6,04 (t, 1H, 3-H), 6,14 (d, 1H, 2-H), 6,28 (dd, 1H, 3'-H), 7,2-7,6 (m, 18H, o- und p-$C_6H_5$), 7,8-8,2 (m, 12H, m-$C_6H_5$); $J_{2,3}$ = 7,1 Hz, $J_{3,4}$ = 6,8 Hz, $J_{4,5}$ = 2,1 Hz, $J_{4,5b}$ = 4,8 Hz, $J_{5a',5b}$ = 11,4 Hz, $J_{1',2'}$ = 3,7 Hz, $J_{2',3'}$ = 9,6 Hz, $J_{3',4'}$ = 9,7 Hz, $J_{4',5'}$ = 9,9 Hz, $J_{5',6a}$ = 4,9 Hz, $J_{6'a,6'b}$ = 12,5 Hz.

MS (FD): m/e = 935 (100%, $M^+ + 1$).

$C_{53}H_{42}O_{16}$ (934,9)

| | | |
|---|---|---|
| Berechnet: | C 68,09 | H 4,53% |
| Gefunden: | C 67,89 | H 4,48% |

*Beispiel 7*

*D-Glucopyranosyl-α(1→5)-D-arabinit (Formel 8)*

Zu einer Lösung von 6,85 g (10 mmol) Methylglucopyranosylarabonat der Formel (3) (Produkt von Beispiel 2) in 250 ml Wasser werden 0,5 g Borsäure eingerührt, danach 70 ml eines stark sauren Ionenaustauschers („Amberlite® IR-120", $H^+$-Form), und auf 0° C gekühlt. Sodann wird im Verlauf einer Stunde eine Lösung von 7,5 g Natriumborhydrid in Wasser (400 ml) unter Rühren und Kühlen zugetropft und nach erfolgter Zugabe noch 1 h weiter gerührt. Durch vorsichtiges Versetzen mit 2N NaOH wird die Lösung auf pH 9-10 gebracht, filtriert, und über eine Ionenaustauschersäule (150 ml „Amberlite® IR-120", $H^+$-Form) gegeben, mit Wasser (ca 21) nachgewaschen, und die vereinigten Eluate werden im Vakuum zu einem Volumen von ca. 100 ml konzentriert. Rühren mit stark basischem Ionenaustauscher für 30 min zur Entfernung noch verbliebener Borsäure, Filtration und nachfolgendes kurzes Rühren mit „Amberlite® IR-120" liefert nach Eindampfen im Vakuum einen Sirup, der beim Digerieren mit Methanol kristallisiert: 5,1 g (81%) Glucopyranosylarabinit der Formel (8); Schmp. 156-158° C, $[\alpha]_D^{20}$ = +98,5° (c = 1, Wasser).

$^1$H-NMR (300 MHz, $D_2O$): δ = 3,43 (t, 1H, 4'-H), 3,58 (dd, 1H, 2'-H), 3,6-4,0 (m, 11H, 1-$H_2$, 2-H, 3-H, 4-H, 5-$H_2$, 3'-H, 5'-H, 6'-$H_2$), 4,95 (d, 1H, 1'H); $J_{1',2'}$ = 3,5 Hz, $J_{2',3'}$ = 9,6 Hz, $J_{3',4'}$ = $J_{4',5'}$ = 9,5 Hz.

$^{13}$C-NMR (75 MHz, $D_2O$): δ = 61,40 (t, C-6'), 63,95 (t, C-1), 69,44 (t, C-5), 70,09 (d, C-2), 70,44 (d, C-4'), 70,90 (d, C-3), 71,05 (d, C-4), 72,36 (d, C-2'), 72,61 (d, C-5'), 73,97 (d, C-3'), 98,99 (d, C-1').

MS (FD): m/e = 337 (100%, $M^+$ + Na), 315 (80%, $M^+ + 1$).

$C_{11}H_{22}O_{10}$ (314,3)

| | | |
|---|---|---|
| Berechnet: | C 42,03 | H 7,06% |
| Gefunden: | C 41,89 | H 7,00% |

*Beispiel 8*

*D-Glucopyranosyl-α(1→5)-D-arabinit-octabenzoat (Formel 9)*

In einem 250 ml-Dreihalskolben mit Rührer werden 3,5 g (10 mmol) Methyl-glucopyranosylarabonat der Formel (3) (Produkt von Beispiel 2) in 70 ml Wasser gelöst, mit 1,4 g (37 mmol) Natriumborhydrid versetzt und 2 h bei Raumtemperatur stehengelassen. Danach wird durch Zugabe von 4,2 ml Aceton das restliche Natriumborhydrid zerstört, Ionenaustauscher „IR-120" ($H^+$-Form) entionisiert, filtriert und das Filtrat zu einem Sirup eingedampft (2,9 g). Benzoylierung in Pyridin (50 ml) mit Benzoylchlorid (32 ml) bei Raumtemperatur (16 h), Einrühren in Eiswasser, Extraktion mit Dichlormethan (3 × 100 ml), Waschen der vereinigten organischen Phasen mit 2N HCl, gesättigter $NaHCO_3$-Lösung und Wasser liefert nach Trocknen ($Na_2SO_4$) und Eindampfen zur Trockne einen Sirup, der durch Elution von einer Silicagel-Säule (500 g) mit Dichlormethan-Äthylacetat (40:1) gereinigt wird: 8,5 g (73%) Octabenzoat der Formel (9) als chromatographisch einheitlichen, farblosen Sirup mit $[\alpha]_D^{24}$ = +79,5° (c = 1, $CHCl_3$).

$^1$H-NMR (300 MHz, $CDCl_3$): δ = 3,92 (dd, 1H, 5-$H_a$), 4,10-4,28 (m, 4H, 5-$H_b$, 5'-H, 6'-$H_2$), 4,61 (dd, 1H, 1-$H_a$), 4,67 (dd, 1H, 1-$H_b$), 5,32 (dd, 1H, 2'H), 5,38 (d, 1H, 1'-H), 5,64 (t, 1H, 4'-H), 5,74 (ddd, 1H, 4-H), 5,95 (ddd, 1H, 2-H), 6,27 (t, 1H, 3'-H), 6,31 (dd, 1H, 3-H), 7,1-7,6 (m, 24H, o- und p-$C_6H_5$), 7,7-8,2 (m, 16H, m-$C_6H_5$); $J_{1a,1b}$ = 11,9, $J_{1a,2}$ = 6,9, $J_{1b,2}$ = $J_{5a,5b}$ = 11,5, $J_{1',2'}$ = 3,7, $J_{2',3'}$ = 10,2, $J_{3',4'}$ = 9,7 Hz.

MS (FD): m/e = 1146 (100%, $M^+$).

$C_{67}H_{54}O_{18}$ (1147,1)

| | | |
|---|---|---|
| Berechnet: | C 70,15 | H 4,75% |
| Gefunden: | C 69,96 | H 4,69% |

Die Ent-O-benzoylierung des Octabenzoats (9) mit Natriummethoxid in Methanol nach Zemplen liefert glatt den Glucopyranosylarabinit der Formel (8) in Ausbeuten von oder über 90%.

**Patentansprüche**

1. Derivate und Reduktionsprodukte der D-Glucopyranosyl-α(1→5)-D-arabonsäure der allgemeinen Formel

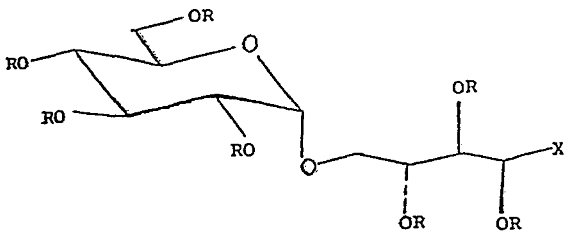

in der X eine

−COOAlkali-,
−COOAlkyl-,
$-CONH_2$- oder
$-CH_2OH$-Gruppe

bedeutet und die Arabonsäurekette auch zum Lactonring geschlossen sein kann und R ein Wasserstoffatom oder einen Acylrest darstellt.

2. Verbindung gemäss Anspruch 1, dadurch gekennzeichnet, dass sie aus dem D-Glucopyranosyl-α(1→5)-D-arabinit der Formel

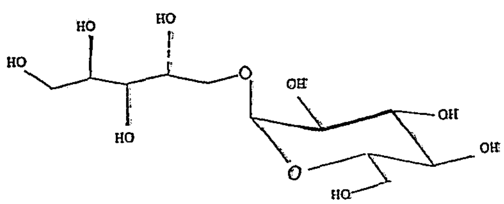

besteht.

3. Verbindung nach Anspruch 1, dadurch gekennzeichnet, dass sie aus dem Kalium-D-Glucopyranosyl-α(1→5)-D-arabonat der Formel

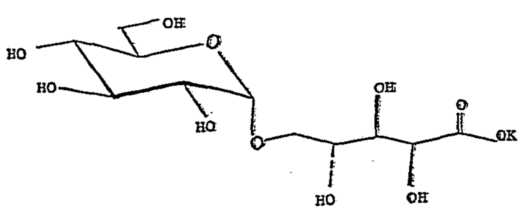

besteht.

4. Verbindung gemäss Anspruch 1, dadurch gekennzeichnet, dass sie aus dem D-Glucopyranosyl-α(1→5)-methyl-D-arabonat der Formel

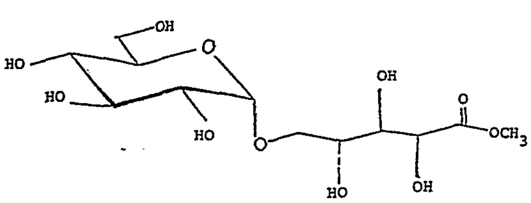

besteht.

5. Verbindung gemäss Anspruch 1, dadurch gekennzeichnet, dass sie aus dem 2,3,4,6-Tetra-O-acetyl-D-glucopyranosyl-α(1→5)-methyl-2,3,4-tri-O-acetyl-D-arabonat der Formel

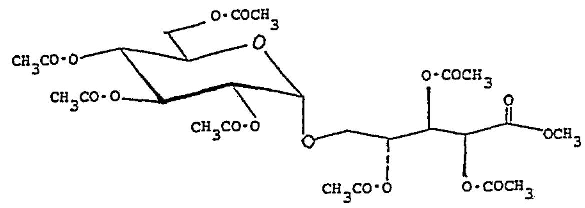

besteht.

6. Verbindung gemäss Anspruch 1, dadurch gekennzeichnet, dass sie aus dem D-Glucopyranosyl-α(1→5)-D-arabonsäureamid der Formel

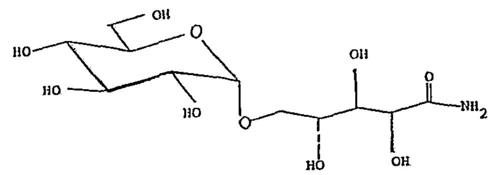

besteht.

7. Verbindung gemäss Anspruch 1, dadurch gekennzeichnet, dass sie aus dem D-Glucopyranosyl-α(1→5)-D-arabonsäure-γ-lacton der Formel

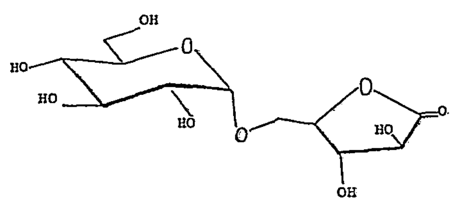

besteht.

8. Verbindung gemäss Anspruch 1, dadurch gekennzeichnet, dass sie aus dem 5-O-(2,3,4,6-Tetra-O-benzoyl-α-D-glucopyranosyl)-2,3-di-O-benzoyl-D-arabonsäure-γ-lacton der Formel

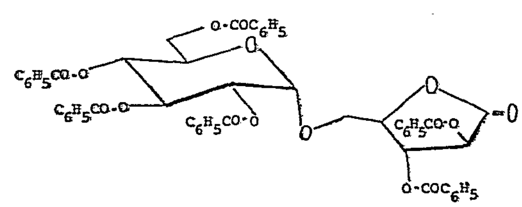

besteht.

9. Verbindung gemäss Anspruch 1, dadurch gekennzeichnet, dass sie aus dem D-Glucopyranosyl-α(1→5)-D-arabinitoctabenzoat der Formel

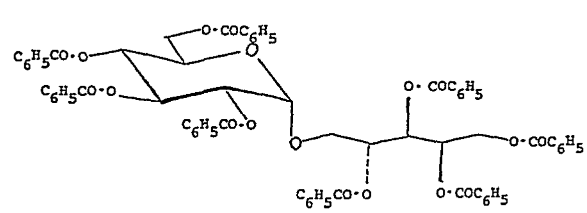

besteht.

10. Verfahren zur Herstellung der Verbindungen gemäss Anspruch 1, dadurch gekennzeichnet, dass man zwecks Gewinnung der Alkalisalze der D-Glucopyranosyl-α-(1→5)-D-arabonsäure Isomaltulose in alkalischer Lösung mit Luft oder Sauerstoff oxidiert und die entstandenen Salze aus

dem Reaktionsgemisch durch direkte Kristallisation isoliert.

11. Verfahren zur Herstellung der Verbindungen gemäss Anspruch 1, dadurch gekennzeichnet, dass man zwecks Gewinnung der Alkylester der D-Glucopyranosyl-α(1→5)-D-arabonsäure die Alkalisalze der genannten Säure, wie sie beim Verfahren des Anspruches 10 anfallen, mit einem Alkanol in Gegenwart eines sauren Ionenaustauschers behandelt.

12. Verfahren zur Herstellung der Verbindungen gemäss Anspruch 1, dadurch gekennzeichnet, dass man zwecks Gewinnung des D-Glucopyranosyl-α(1→5)-D-arabonsäure-γ-lactons die Alkalisalze der genannten Säure, wie sie beim Verfahren des Anspruches 10 anfallen, mit einem sauren Ionenaustauscher in wässeriger Lösung behandelt.

13. Verfahren zur Herstellung der Verbindungen gemäss Anspruch 1, dadurch gekennzeichnet, dass man zwecks Gewinnung des D-Glucopyranosyl-α(1→5)-D-arabonsäureamids einen Alkylester der D-Glucopyranosyl-α(1→5)-D-arabonsäure in Methanol mit trockenem Ammoniak behandelt.

14. Verfahren zur Herstellung der Verbindungen gemäss Anspruch 1, dadurch gekennzeichnet, dass man zwecks Gewinnung von D-Glucopyranosyl-α(1→5)-D-arabinit einen beim Verfahren des Anspruches 11 anfallenden D-Glucopyranosyl-α(1→5)-D-arabonsäurealkylester bzw. dessen Heptaacylat oder das beim Verfahren des Anspruches 12 anfallende D-Glucopyranosyl-α(1→5)-D-arabonsäure-γ-lacton bzw. dessen Hexabenzoat mit einem komplexen Metallhydrid oder mit katalytisch aktiviertem Wasserstoff reduziert und den entstandenen Glucopyranosyl-α(1→5)-D-arabinit durch Kristallisation isoliert.

15. Verfahren zur Herstellung der Verbindungen gemäss Anspruch 1, dadurch gekennzeichnet, dass man zwecks Gewinnung des 2,3,4,6-Tetra-O-acetyl-D-glucopyranosyl-α(1→5)-methyl-2,3,4-tri-O-acetyl-D-arabonats das D-Glucopyranosyl-α(1→5)-methyl-D-arabonat mit Essigsäureanhydrid in einem wasserfreien Lösungsmittel, vorzugsweise Pyridin, acetyliert.

16. Verfahren zur Herstellung der Verbindungen gemäss Anspruch 1, dadurch gekennzeichnet, dass man zwecks Gewinnung des 5-O-(2,3,4,6-Tetra-O-benzoyl-α-D-glucopyranosyl)-2,3-di-O-benzoyl-D-arabonsäure-γ-lactons das D-Glucopyranosyl-α(1→5)-D-γ-lacton mit Benzoylchlorid in einem wasserfreien Lösungsmittel, vorzugsweise Pyridin, benzoyliert.

17. Verfahren zur Herstellung der Verbindungen gemäss Anspruch 1, dadurch gekennzeichnet, dass man zwecks Gewinnung des D-Glucopyranosyl-α(1→5)-D-arabinitoctabenzoats den D-Glucopyranosyl-α(1→5)-D-arabinit mit Benzoylchlorid in einem wasserfreien Lösungsmittel, vorzugsweise Pyridin, benzoyliert.

18. Verwendung der Verbindungen des Anspruches 1 als Zwischenprodukte für chemische Synthesen, insbesondere für die Herstellung von oberflächenaktiven Stoffen.

19. Verwendung des D-Glucopyranosyl-α(1→5)-D-arabinits für sich allein oder im Gemisch mit Süssungsmitteln, wie Palatinit, Saccharose oder solchen Mitteln, die eine höhere Süsskraft als Saccharose haben, als Zuckeraustauschstoff für Nahrungs-, Genussmittel und Getränke.

## Claims

1. Derivatives and reduction products of D-glucopyranosyl-α(1→5)-D-arabonic acid having the general formula

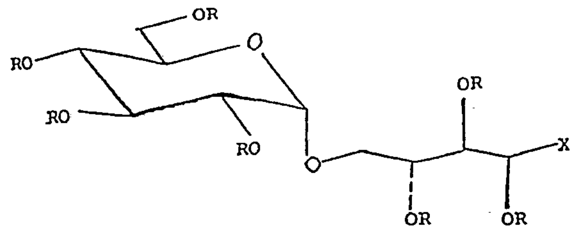

in which X represents a

- $-COO$ alkali,
- $-COO$ alkyl,
- $-CONH_2$ or
- $-CH_2OH$ group

and the arabonic acid chain can also be closed to form the lactone ring and R represents a hydrogen atom or an acyl residue.

2. A compound according to Claim 1, characterized in that it is composed of D-glucopyranosyl-α(1→5)-D-arabinite having the formula

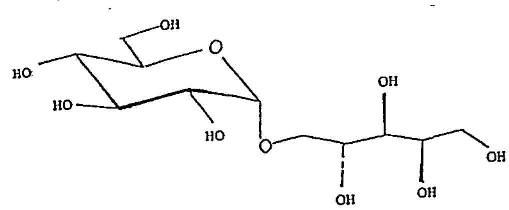

3. A compound according to Claim 1, characterized in that it is composed of potassium-D-glucopyranosyl-α(1→5)-D-arabonate having the formula

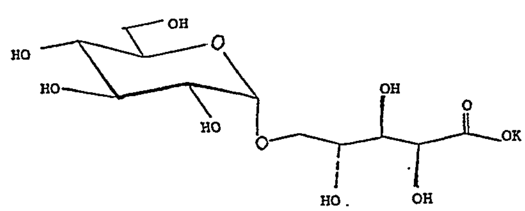

4. A compound according to Claim 1, characterized in that it is composed of D-glucopyranosyl-α(1→5)-methyl-D-arabonate having the formula

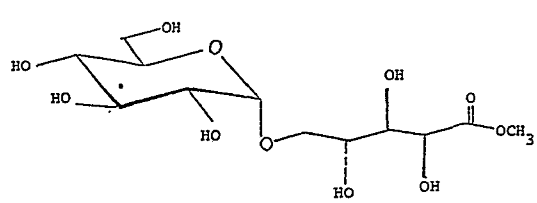

5. A compound according to Claim 1, characterized in that it is composed of 2,3,4,6-tetra-O-acetyl-D-glucopyranosyl-α(1→5)-methyl-2,3,4-tri-O-acetyl-D-arabonate having the formula

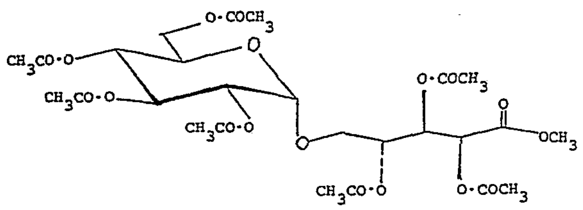

6. A compound according to Claim 1, characterized in that it is composed of D-glucopyranosyl-α(1→5)-D-arabonic acid amide having the formula

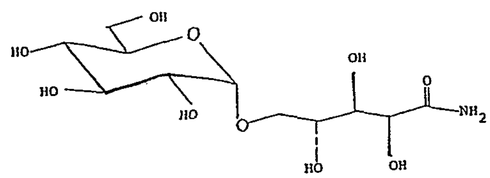

7. A compound according to Claim 1, characterized in that it is composed of D-glucopyranosyl-α(1→5)-D-arabonic acid-γ-lactone having the formula

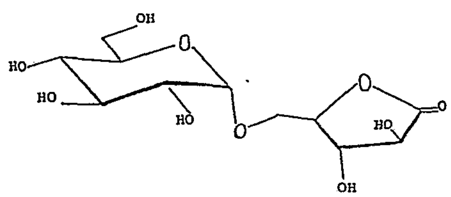

8. A compound according to Claim 1, characterized in that it is composed of 5-O-(2,3,4,6-tetra-O-benzyl-α-D-glucopyranosyl)-2,3-di-O-benzoyl-D-arabonic acid-γ-lactone having the formula

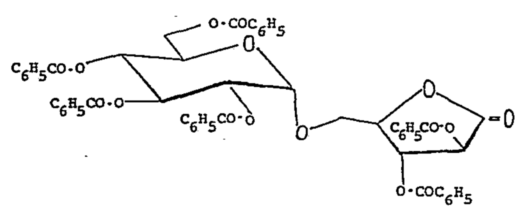

9. A compound according to Claim 1, characterized in that it is composed of D-glucopyranosyl-α(1→5)-D-arabinite-octabenzoate having the formula

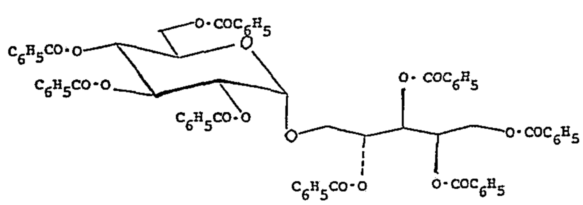

10. A method of preparing the compounds according to Claim 1, characterized in that, for the purpose of obtaining alkali salts of D-glucopyranosyl-α(1→5)-D-arabonic acid, isomaltulose is oxidised in alkaline solution with air or oxygen, and the resultant salts are isolated from the reaction mixture by means of direct crystallization.

11. A method of preparing the compounds according to Claim 1, characterized in that, for the purpose of obtaining alkyl esters of D-glucopyranosyl-α(1→5)-D-arabonic acid, the alkali salts of the aforesaid acids, as are formed in the method of Claim 10, are treated with an alcohol in the presence of an acidic ion exchanger.

12. A method of preparing the compounds according to Claim 1, characterized in that, for the purpose of obtaining D-glucopyranosyl-α(1→5)-D-arabonic acid-γ-lactone, the alkali salts of the aforesaid acids, as are formed in the method of Claim 10, are treated with an acidic ion exchange in aqueous solution.

13. A method of preparing the compounds according to Claim 1, characterized in that, for the purpose of obtaining D-glucopyranosyl-α(1→5)-D-arabonic acid amide, an alkyl ester of D-glucopyranosyl-α(1→5)-D-arabonic acid in methanol is treated with dry ammonia.

14. A method of preparing the compounds according to Claim 1, characterized in that, for the purpose of obtaining D-glucopyranosyl-α(1→5)-D-arabinite, a D-glucopyranosyl-α(1→5)-D-arabinite acid alkyl ester formed in the method of Claim 11, or its heptaacylate, or the D-glucopyranosyl-α(1→5)-D-arabonic acid-γ-lactone formed in the method of Claim 12, or its hexabenzoate, is reduced with a complex metallic hydride or with catalytically activated hydrogen; and the resultant glucopyranosyl-α(1→5)-D-arabinite is isolated by means of crystallization.

15. A method of preparing the compounds according to Claim 1, characterized in that, for the purpose of obtaining 2,3,4,6-tetra-O-acetyl-D-glucopyranosyl-α(1→5)-methyl-2,3,4-tri-O-acetyl-D-arabonate, the D-glucopyranosyl-α(1→5)-methyl-D-arabonate is acetylated with acetic anhydride in an anhydrous solvent, preferably pyridine.

16. A method of preparing the compounds according to Claim 1, characterized in that, for the purpose of obtaining 5-O-(2,3,4,6-tetra-O-benzoyl-α-D-glucopyranosyl)-2,3-di-O-benzoyl-D-arabonic acid-γ-lactone, D-glucopyranosyl-α(1→5)-D-γ-lactone is benzoylated with benzoyl chloride in an anhydrous solvent, preferably pyridine.

17. A method of preparing the compounds according to Claim 1, characterized in that, for the purpose of obtaining D-glucopyranosyl-α-(1→5)-D-arabinite-octabenzoate, the D-glucopyranosyl-α(1→5)-D-arabinite is benzoylated with benzoyl chloride in an anhydrous solvent, preferably pyridine.

18. The use of the compounds of Claim 1 as intermediate products for chemical syntheses, in particular for the production of surface-active substances.

19. The use of D-glucopyranosyl-α(1→5)-D-arabinite by itself or mixed with sweetening

agents, e.g. palatinite, saccharose or such agents which have a greater sweetening power than saccharose, as a sugar substitute for foodstuffs, stimulants and beverages.

## Revendications

1. Dérivés et produits de réduction de l'acide D-glucopyrannosyl-alpha-(1-5)-D-arabonique de formule générale

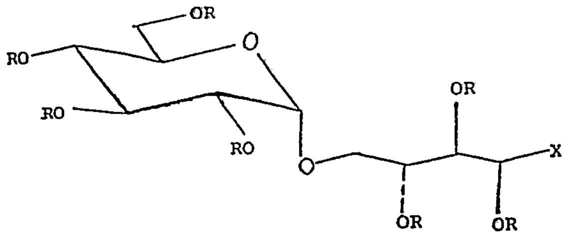

dans laquelle X représente un groupe

- $-COO$ alcalin,
- $-COO$ alcoyle,
- $-CONH_2$ ou
- $-CH_2OH$

et la chaîne de l'acide arabonique peut aussi être cyclisée en une lactone, tandis que R représente un atome d'hydrogène ou un reste d'acyle.

2. Combinaison suivant la revendication 1, caractérisée en ce qu'elle est constituée par le D-arabinite-D-glucopyrannosyl-alpha-(1-5) de la formule

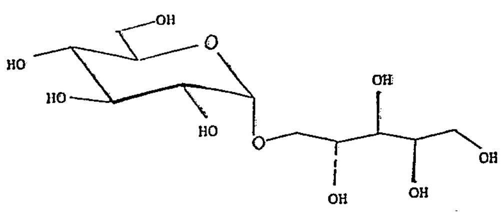

3. Combinaison suivant la revendication 1, caractérisée en ce qu'elle est constituée par le D-arabonate potassium-D-glucopyrannosyl-alpha-(1-5) de la formule

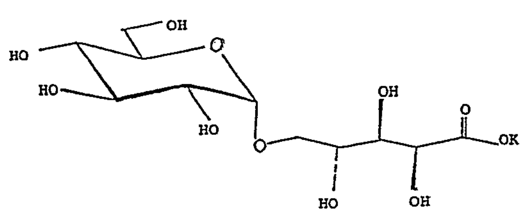

4. Combinaison suivant la revendication 1, caractérisée en ce qu'elle est constituée par le D-arabonate-D-glucopyrannosyl-alpha-(1-5)-méthyle de la formule

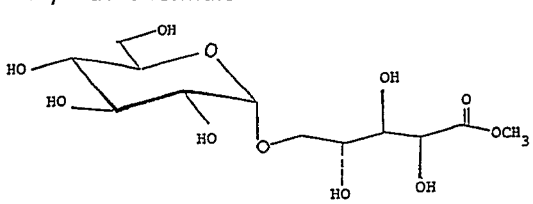

5. Combinaison suivant la revendication 1, caractérisée en ce qu'elle est constituée par le D-arabonate 2,3,4,6-tétra-O-acétyle-D-glucopyrannosyl-alpha-(1-5)-méthyle-2,3,4,6-tri-O-acétyle de la formule

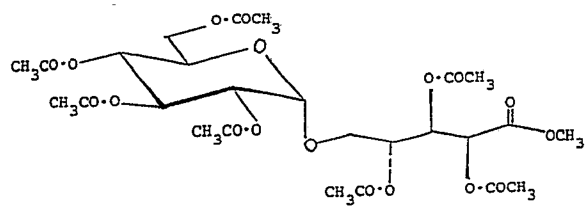

6. Combinaison suivant la revendication 1, caractérisée en ce qu'elle est constituée par l'amide de l'acide D-glucopyrannosyl-alpha-(1-5)-D-arabonique de la formule

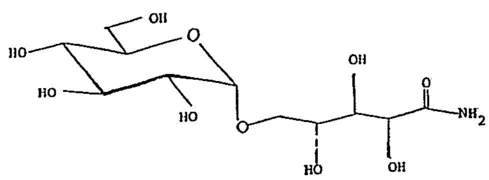

7. Combinaison suivant la revendication 1, caractérisée en ce qu'elle est constituée par la gamma-lactone de l'acide D-glucopyrannosyl-alpha-(1-5)-D-arabonique de la formule

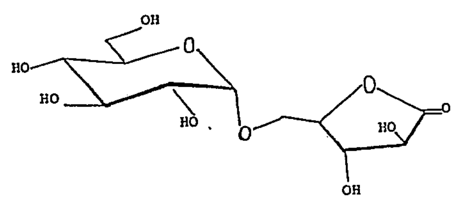

8. Combinaison suivant la revendication 1, caractérisée en ce qu'elle est constituée par la gamma-lactone de l'acide 5-O-(2,3,4,6-tétra-O-benzoyle-alpha-D-glucopyrannosyl)-2,3-di-O-benzoyle-D-arabonique de la formule

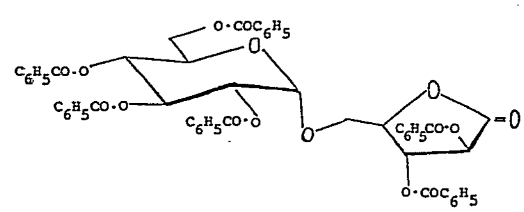

9. Combinaison suivant la revendication 1, caractérisée en ce qu'elle est constituée par l'octabenzoate-D-arabinite-D-glucopyrannosyl-alpha-(1-5) de la formule

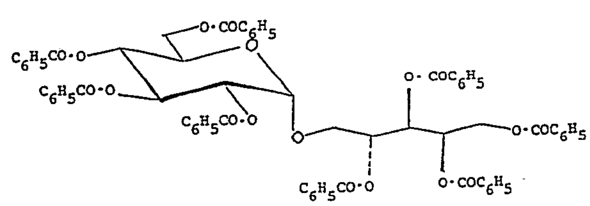

10. Procédé pour la préparation des combinaisons suivant la revendication 1, caractérisé en ce

que, pour obtenir les sels alcalins de l'acide D-glucopyrannosyl-alpha-(1-5)-D-arabonique, on oxyde de l'isomaltulose en solution alcaline avec de l'air ou de l'oxygène et on isole du mélange réactionnel les sels obtenus, par cristallisation directe.

11. Procédé pour la préparation des combinaisons suivant la revendication 1, caractérisé en ce que, pour obtenir les esters alcoyles de l'acide D-glucopyrannosyl-alpha-(1-5)-D-arabonique, les sels alcalins de l'acide indiqué, tels qu'ils sont obtenus avec le procédé de la revendication 10, sont traités avec un alcanol, en présence d'un échangeur d'ions acide.

12. Procédé pour la préparation des combinaisons suivant la revendication 1, caractérisé en ce que, pour obtenir la gamma-lactone de l'acide D-glucopyrannosyl-alpha-(1-5)-D-arabonique, les sels alcalins de l'acide indiqué, tels qu'ils sont obtenus avec le procédé de la revendication 10, sont traités avec un échangeur d'ions acide dans une solution aqueuse.

13. Procédé pour la préparation des combinaisons suivant la revendication 1, caractérisé en ce que, pour obtenir l'amide de l'acide D-glucopyrannosyl-alpha-(1-5)-D-arabonique, on traite un ester alcoyle de l'acide D-glucopyrannosyl-alpha-(1-5)-D-arabonique dans le méthanol avec de l'ammoniac sec.

14. Procédé pour la préparation des combinaisons suivant la revendication 1, caractérisé en ce que, pour obtenir du D-arabinite D-glucopyrannosyl-alpha-(1-5), on réduit un ester alcoyle de l'acide D-glucopyrannosyl-alpha-(1-5)-D-arabonique, obtenu avec le procédé de la revendication 11, et/ou son heptaacylate ou la gamma-lactone de l'acide D-glucopyrannosyl-alpha-(1-5)-D-arabonique, obtenu avec le procédé de la revendication 12 et/ou son hexabenzoate, avec un hydrure métallique complexe ou avec de l'hydrogène activé catalytiquement et on isole par cristallisation le D-arabinite glucopyrannosyl-alpha-(1-5) obtenu.

15. Procédé pour la préparation des combinaisons suivant la revendication 1, caractérisé en ce que, pour obtenir le D-arabonate-2,3,4,6-tétra-O-acétyle-D-glucopyrannosyl-alpha-(1-5)-méthyle-2,3,4-tri-O-acétyle, on acétylise le D-arabonate D-glucopyrannosyl-alpha-(1-5) méthyle avec un anhydride de l'acide acétique dans un solvant sec, de préférence de la pyridine.

16. Procédé pour la préparation des combinaisons suivant la revendication 1, caractérisé en ce que, pour obtenir la gamma-lactone de l'acide 5-O-(2,3,4,6-tétra-O-benzoyle-alpha-D-glucopyrannosyl)-2,3-di-O-benzoyle-D-arabonique, on benzoylise la D-gamma-lactone D-glucopyrannosyl-alpha-(1-5) avec du chlorure de benzoyle, dans un solvant sec, de préférence de la pyridine.

17. Procédé pour la préparation des combinaisons suivant la revendication 1, caractérisé en ce que, pour obtenir l'octabenzoate-D-glucopyrannosyl-alpha-(1-5)-D-arabinite, on benzoylise le D-arabinite-D-glucopyrannosyl-alpha-(1-5) avec du chlorure de benzoyle, dans un solvant sec, de préférence de la pyridine.

18. Utilisation des combinaisons de la revendication 1 comme produits intermédiaires pour des synthèses chimiques, spécialement pour la préparation de substances tensio-actives.

19. Utilisation du D-arabinite-D-glucopyrannosyl-alpha-(1-5) seul ou mélangé à des édulcorants, comme le palatinite, le saccharose ou d'autres moyens de pouvoir sucrant supérieur à celui du saccharose, comme substance de substitution du sucre pour des produits alimentaires, des produits d'épicerie fine et des boissons.